# EUROPEAN PATENT APPLICATION

(11) **EP 2 671 504 A2**
(43) Date of publication of application: **11.12.2013**
(21) Application number: 13169884.7
(22) Date of filing: 30.05.2013
(51) Int. Cl.: A61B 1/24

(54) **Multifunction wand for an intra-oral imaging system**

(30) Priority: 06.06.2012 US 201213490289
(71) Applicant: ORMCO CORPORATION, Orange, CA 92867 (US)
(72) Inventor: Dillon, Robert F, Bedford, NH New Hampshire 03110 (US); Andreiko, Craig A, Alta Loma, CA California 91737 (US); Krohg, Olaf N, Topsfield, MA Massachusetts 01983 (US)
(74) Representative: Findlay, Alice Rosemary

(57) **Abstract**

A wand (104) having a housing (206, 302) is coupled to an intra-oral imaging system (102). The wand (104) comprises a first device and a second device coupled to the housing (206, 302). The first device is an intra-oral imaging device (106) for capturing images of a patient's teeth, and the second device provides an additional function.

## Description

The disclosure relates to a multifunction wand for an intra-oral imaging system.

An intra-oral imaging system is a diagnostic equipment that allows a dental practitioner to see the inside of a patient's mouth and display the topographical characteristics of teeth on a display monitor. Certain three-dimensional (3D) intra-oral imagers may be comprised of an intra-oral camera with a light source. The 3D intra-oral imager may be inserted into the oral cavity of a patient by a dental practitioner. After insertion of the intra-oral imager into the oral cavity, the dental practitioner may capture images of visible parts of the teeth and the gingivae.

The 3D intra-oral imager may be fabricated in the form of a slender rod that is referred to as a wand or a handpiece. The wand may be approximately the size of a dental mirror with a handle that is used in dentistry. The wand may have a built-in light source and a video camera that may achieve an imaging magnification, ranging in scale from 1 to 40 times or more. This allows the dental practitioner to discover certain types of details and defects of the teeth and gums. The images captured by the intra-oral camera may be displayed on a television or a computer monitor.

The wand may be attached or linked to a computer and a display monitor. The wand, the computer, and the display monitor may all be placed in the proximity of the patient before the dental practitioner places the tip of the wand inside the oral cavity of the patient and starts acquiring images. The acquired images may be displayed on the display monitor and may also be saved on a storage device. Furthermore, the acquired images may be transmitted to a remote computational device for additional processing.

A wand having a housing is coupled to an intra-oral imaging system. The wand comprises a first device and a second device coupled to the housing. The first device is an intra-oral imaging device for capturing images of a patient's teeth, and the second device provides an additional function.

In certain embodiments, the second device is a camera for capturing images of the patient's face.

Alternatively or additionally, the second device is a light emitting diode (LED) curing light for hardening sealants.

In a further embodiment, the LED curing light is positioned to emit light through a tip of wand, wherein a curing light control is positioned on a handle of the wand, and wherein the curing light control controls operation of the LED curing light.

Alternatively or additionally, the second device is a microphone for capturing oral instructions of a patient or a dental practitioner.

Alternatively or additionally, the second device is an ultrasound device to capture ultrasound imagery of the patient's oral cavity.

Alternatively or additionally, the second device is a laser emitter device that generates photoacoustic waves greater than 1200 nanometers in wavelength to turbulently clean interiors of root and lateral canal systems or cause cell lysis and dissolution of inorganics in biotic systems.

Alternatively or additionally, the second device is a high-energy light emitting device for detecting caries.

In certain embodiments, the intra-oral imaging device is a first camera, and the second device is a second camera for capturing images of the patient's face. The wand further comprises a tip, wherein the first camera for capturing the images of the patient's teeth is positioned to image through the tip. The wand also comprises a handle for holding the wand, wherein the second camera for capturing the images of the patient's face is positioned on the handle.

In additional embodiments, the wand further comprises a camera control positioned on the handle, wherein the camera control controls operation of the second camera.

In certain embodiments, the second camera is at least one of a still camera and a video camera.

In further embodiments, the wand is positioned to capture a sequence of images of a patient's face, wherein the sequence of images are processed to generate a two-dimensional image of the patient's face.

In yet further embodiments, the wand is positioned to capture a sequence of images of a patient's face, wherein the sequence of images are processed to generate a three-dimensional image of the patient's face.

In certain embodiments, the generated three-dimensional image of the patient's face are combined with at least one of X-ray imagery, dental impression, and intra-oral imagery, to generate a rotatable three-dimensional model of the patient's face with an embedded three-dimensional model of the patient's teeth.

In additional embodiments, the intra-oral imaging device is a first camera, and the second device is a second camera for capturing images of the patient's face. The wand further comprises: a light emitting diode (LED) curing light for hardening sealants; a microphone for capturing oral instructions of a patient or a dental practitioner; an ultrasound device to capture ultrasound imagery of the patient's oral cavity; a laser emitter device that generates photoacoustic waves greater than 1200 nanometers in wavelength to turbulently clean interiors of root and lateral canal systems or cause cell lysis and dissolution of inorganics in biotic systems; and a high-energy light emitting device for detecting caries.

The invention will now be further described by way of example with reference to the accompanying drawings, in which:

FIG. 1 illustrates a block diagram of a computing and imaging environment that includes an intra-oral imaging system having a multifunction wand with an intra-oral imaging sensor for capturing images for a patient's teeth and a camera for imaging the patient's face, in accordance with certain embodiments;

FIG. 2 illustrates an exemplary intra-oral imaging system in which the multifunction wand is included, in accordance with certain embodiments;

FIG. 3 illustrates the multifunction wand in which the intra-oral imaging sensor for capturing images for a patient's teeth and the camera for imaging the patient's face are included, in accordance with certain embodiments;

FIG. 4 illustrates a diagram that shows various elements of the multifunction wand, in accordance with certain embodiments;

FIG. 5 illustrates another diagram that shows various elements of the multifunction wand, in accordance with certain embodiments;

FIG. 6 illustrates a block diagram that shows an operator holding the multifunction wand via a handle, in accordance with certain embodiments;

FIG. 7 illustrates an operator capturing a sequence of images of a patient's face from various positions and orientations of the multifunction wand, in accordance with certain embodiments;

FIG. 8 illustrates a block diagram that shows how three dimensional images of the face of a patient are generated via photogrammetry techniques, in accordance with certain embodiments; and

FIG. 9 illustrates a block diagram that shows how a rotatable 3D model of a patient's face with embedded three-dimensional model of teeth is generated, in accordance with certain embodiments;

FIG. 10 illustrates exemplary operations performed in accordance with certain embodiments; and

FIG. 11 illustrates a block diagram of a computational system that shows certain elements of an intra-oral imaging system, in accordance with certain embodiments.

Certain embodiments are disclosed that provide a method, apparatus, and a computer readable storage medium for the operation of a two-dimensional (2D) color camera that is incorporated into an intra-oral imaging system. The intra-oral imaging system captures both intra-oral images of the teeth of a patient and also captures one or more images of the face of the patient.

In certain embodiments, the camera may be used to capture one or more 2D images of the patient's entire face or parts of the face, such as the mouth. In certain embodiments, a plurality of 2D images captured from different orientations may be used to generate a 3D image of the entire face via photogrammetry techniques.

The image of the face, whether one image for a 2D view or several images for a 3D view, may be used in dentistry and in other areas. For example, the images may be used for determining tooth shade, facial alignment (such as face bow, facial asymmetry, and profile) and general facial features for treatment planning, and for matching digital impressions and other digital data to a medical record that may be identified by the patient's image.

In certain embodiments, when combined with images from an X-ray system and/or a digital impression, the images from the camera may be used for treatment planning, and for showing imagery acquired both before and after treatment for patient education.

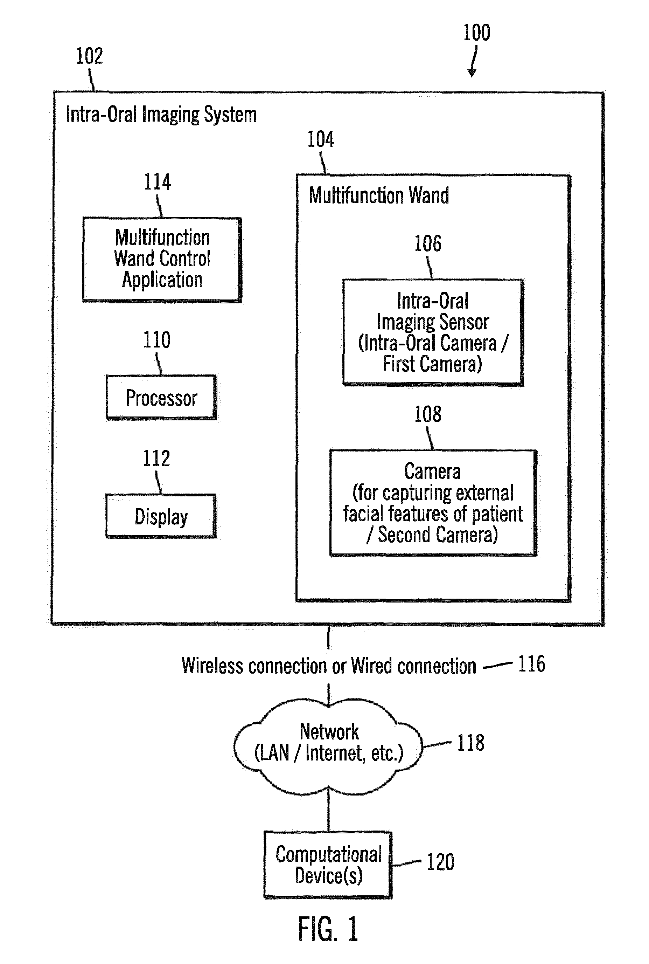

FIG. 1 illustrates a block diagram of a computing and imaging environment 100 that includes an intra-oral imaging system 102 having a multifunction wand 104 with an intra-oral imaging sensor 106 for capturing images of a patient's teeth, and a camera 108 for imaging the patient's face, in accordance with certain embodiments.

The intra-oral imaging system 102 is comprised of a processor 110, a display 112, a multifunction wand 104, and multifunction wand control application 114. The intra-oral imaging system 102 may be coupled via a wired or wireless connection 116 over a network 118 to one or more computational devices 120. The computational devices 120 may include any suitable computational device such as a personal computer, a server computer, a mini computer, a mainframe computer, a blade computer, a tablet computer, a touchscreen computing device, a telephony device, a cell phone, a mobile computational device, etc., and some of the computational devices may provide web services or cloud computing services. The network 118 may comprise any suitable network known in the art such as a local area network, an intranet, the Internet, a storage area network, etc.

A dental practitioner may hold the multifunction wand 104 inside a patient's oral cavity. An optical source coupled to the multifunction wand 104 may illuminate the oral cavity and the intra-oral imaging sensor 106 may be used to capture a plurality of digital images of structures in the oral cavity, such as the patient's teeth, gingivae, and/or palate, and other structures, such as fillings, braces, etc. In certain embodiments the intra-oral imaging sensor 106 may comprise an intra-oral camera.

The dental practitioner may also move the multifunction wand 104 around the patient's face to capture a plurality of images of the patient's face from different angles. For example, in certain embodiments a plurality of frontal, lateral, and other views of the patient's face may be captured.

The operation of the multifunction wand 104 may be controlled by the multifunction wand control application 114 that may be implemented in certain embodiments in software, hardware, firmware or any combination thereof. The multifunction wand control application 114 may process the images acquired by the intra-oral imaging sensor 106 and the camera 108 and display the images on the display 112, where the display may comprise a touchscreen display. In certain alternative embodiments additional or alternative processing of the images acquired by the intra-oral imaging sensor 106 and the camera 108 may be performed over the network 118 by the computational device 120, and the multifunction wand control application 114 may then display the processed images on the display 112.

Therefore, FIG. 1 illustrates certain embodiments in which an intra-oral imaging system 102 is augmented with a multifunction wand 104 that includes at least an intra-oral imaging sensor 106 for capturing intra-oral images, and a camera 108 for capturing external facial features of a patient.

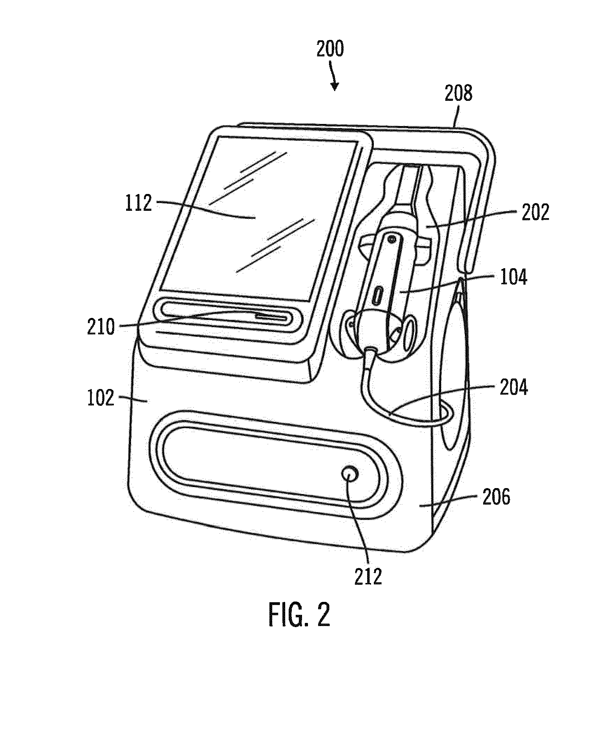

FIG. 2 illustrates a view 200 of an exemplary intra-oral imaging system 102 in which the multifunction wand 104 having the intra-oral imaging sensor 106 and the camera 108 are included, in accordance with certain embodiments. It should be noted that intra-oral imaging system 102 is exemplary and other intra-oral imaging systems may be used in alternative embodiments.

The intra-oral imaging system 102 may include a multifunction wand 104 having the intra-oral imaging sensor 106 and the camera 108. The multifunction wand 104 is small and light weight for use by dental practitioners, and the intra-oral imaging process is fast and relatively simple to use, allowing the imaging of both arches and bites to be accomplished rapidly, such that a digital model of the imaged areas may be viewed on the display 112, where in certain embodiments the display 112 is a touchscreen display.

The intra-oral imaging system 102 may include a wand storage area 202 in which the multifunction wand 104 may be stored. The multifunction wand 104 may be extensibly coupled via a cord 204 to the housing 206 of the intra-oral imaging system 102.

The intra-oral imaging system 102 may include a handle 208 that may be used for carrying the intra-oral imaging system 102 from one location to another. The handle 208 may also be referred to as a carrying handle.

In addition to the handle 208, the display 112, the multifunction wand 104, and the housing 206, the intra-oral imaging system 102 includes a power button 212 that is located on the front face of the intra-oral imaging system 102. The power button 212 may be used to switch the intra-oral imaging system 102 on and off. Additionally, light emitting diode (LED) based indicators 210 may indicate one or more status related to the operational state of the intra-oral imaging system 102.

Therefore, FIG. 2 illustrates certain embodiments in which an intra-oral imaging system 102 includes a multifunction wand 104 that includes an intra-oral imaging sensor 106 and a camera 108.

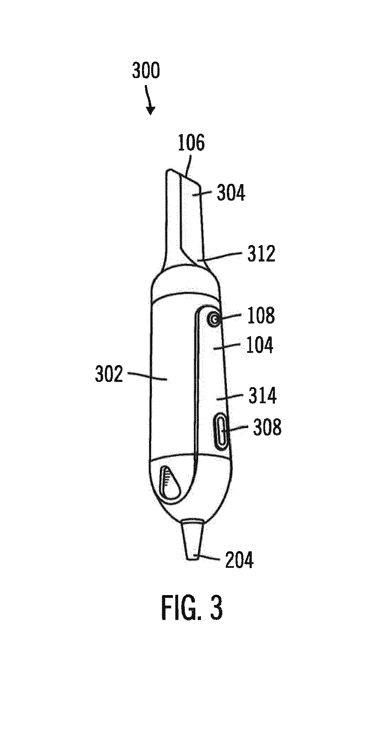

FIG. 3 illustrates a view 300 of the multifunction wand 104 in which the intra-oral imaging sensor 106 for capturing images for a patient's teeth and the camera 108 for imaging the patient's face are included, in accordance with certain embodiments. Components of the wand 104 may be wholly or partially enclosed within a housing 302 that protects the optical components of the wand 104 from dust and debris to maintain measurement accuracy.

The tip 304 is the portion of the wand 104 that is inserted into a patient's mouth. The intra-oral imaging sensor 106 may be embedded within the tip 304 of the wand. The tip 304 of the wand 104 may include an optical window made of biocompatible, transparent material that may be either plastic or glass. The optical window may be mounted into the plastic tip housing such that no sharp corners or edges contact human tissue. The light from the optical source is transmitted through the optical window, and the imaging device 106 captures images of the structures of the oral cavity through the optical window. It should be emphasized that the wand tip 304 is designed to be long enough to reach the back teeth of a typical patient.

The wand 104 has a molded area 308 in which there are keypad buttons and controls to traverse through items from the graphical user interface displayed on the display 112 and for controlling various elements of the wand 104 such as the intra-oral imaging sensor 106 and the camera 108. In certain embodiments, the tip 304 of the wand 104 is covered with a disposable molded plastic sheath that snaps on and off the wand 104. The disposable molded plastic sheath may be transparent and may have a mirror.

The end comprising the tip 304 of the wand 104 may be referred to as the distal end 312 of the wand and the end to which the cord 204 is extensibly coupled may be referred to as the proximal end 314 of the wand 104.

Therefore, FIG. 3 illustrates certain embodiments in which a multifunction wand 104 includes at least an intra-oral imaging sensor 106 and a camera 108.

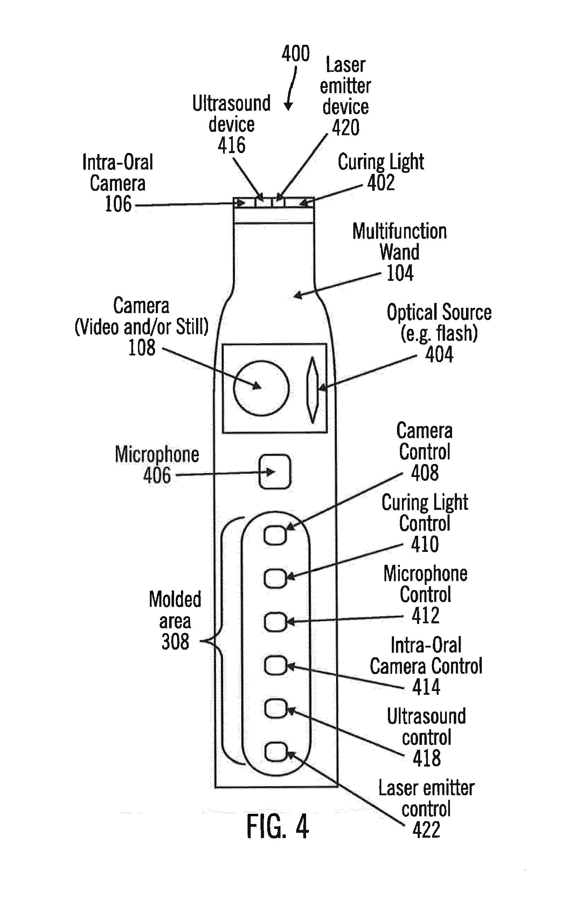

FIG. 4 illustrates a diagram 400 that shows various elements of the multifunction wand 104, in accordance with certain embodiments.

The multifunction wand 104 has an intra-oral camera 106 and a curing light 402 positioned at the tip of the intra-oral camera 106. The curing light 402 is a type of dental equipment that is used to cure (i.e. harden) resin based composites. The curing light 402 may be used on several different dental materials that are curable (i.e., can be hardened) by light. The light used may fall under the visible blue light spectrum. Exemplary curing lights may be of various types, such as tungsten halogen, light-emitting diode (LED), plasma arc curing (PAC), and laser.

The camera 108 included in the multifunction wand 104 may be a video camera or a still camera. The camera 108 may be a color camera or a grayscale image capturing camera. Associated with the camera 108 is an optical source 404, such as a flash. The flash may be triggered when images are captured for securing better quality still images in comparison to cameras that do not have an associated flash.

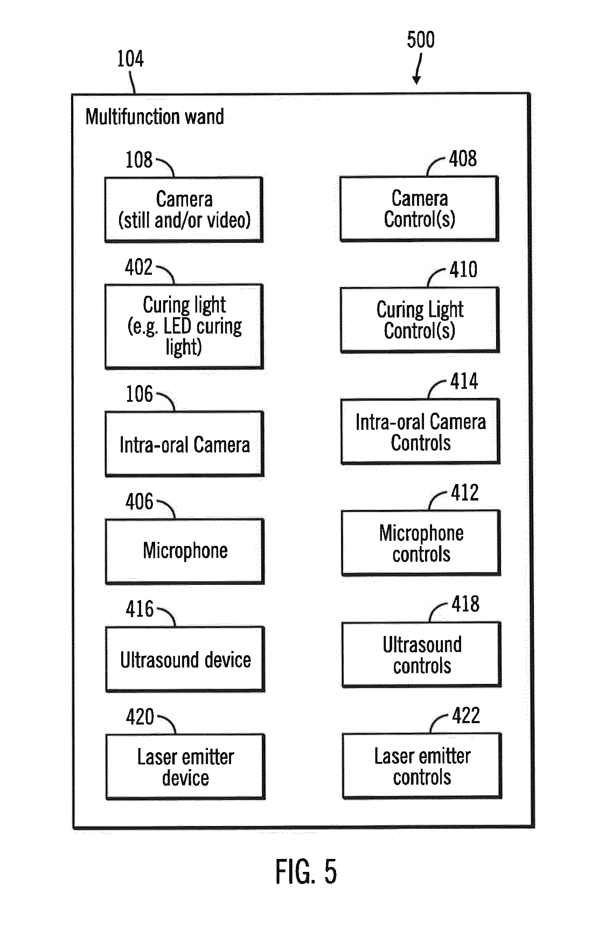

The multifunction wand 104 may also include a microphone 406 embedded in the housing of the multifunction 104. The microphone 406 may be used to capture instructions conveyed via speech by the dental practitioner or the patient. It may be noted from FIG. 5 that the multifunction wand 104 may also include an ultrasound device 416 to capture ultrasound imagery of the patient's oral cavity, and a laser emitter device 420 that generates photoacoustic waves greater than 1200 nanometers in wavelength to turbulently clean interiors of root and lateral canal systems or cause cell lysis and dissolution of inorganics in biotic systems. The molded area 308 in the multifunction wand 104 may include control knobs, buttons, switches, etc., referred to as camera control 408, curing light control 410, microphone control 412, intra-oral camera control 414, ultrasound control 418, and laser emitter control 422, for controlling the operations of the camera 108, the curing light 402, the microphone 406, the intra-oral camera 106, the ultrasound device 416, and the laser emitter device 420 respectively. In certain embodiments the controls 408, 410, 412, 414, 418, 422 may be implemented differently or may be more or fewer in number. In certain alternative embodiments, control for a plurality of components of the multifunction wand 104 may be performed by a single control knob, button, switch, etc.

FIG. 5 illustrates a block diagram 500 that shows various elements of the multifunction wand 104 in accordance with certain embodiments. In FIG. 5 it is shown that the multifunction wand 104 has a camera (still and/or video) 108 that is controlled via camera controls 408, a curing light 402 that is controlled via curing light controls 410, an intra-oral camera 106 that is controlled via intra-oral camera controls 414, a microphone 406 that is controlled via microphone controls 412, and ultrasound device 416 that is controlled by ultrasound controls 418, and a laser emitter 420 that emits wavelengths that are greater than 1200 nanometers where the laser emitter 420 is controlled by laser emitter controls 422. The controls 408, 410, 412, 414, 418, 422 may be used not only to start and stop the operation of the corresponding device in the multifunction wand, but may also be used to adjust other parameters of operation. For example, the camera control 408 may be used to focus the camera, and the microphone control 412 may be used to adjust the volume sensitivity of the microphone 406 for capturing speech. In certain embodiments, the different devices incorporated in the multifunction wand 104 have to be operated at different times and in some embodiments two or more devices incorporated in the multifunction wand 104 may be operated at the same time. For example, the microphone 406 and the still camera 108 may be operated substantially simultaneously, whereas the intra-oral camera 106 may and the still camera 108 may be operated at different times.

In certain embodiments, additional devices beyond those shown in FIGS. 4-5 may be included in the multifunction wand 104. For example, in certain embodiments the multifunction wand 104 may include a high-energy light emitting device for detecting caries, and associated controls.

Not all of the devices 108, 402, 106, 406, 416, 420 shown in FIGS. 4-5 have to be present in the multifunction wand. In certain embodiments, the multifunction wand 104 may have an intra-oral camera 106 along with one other device that performs a function that is different from intra-oral scanning. The additional function that is different from intra-oral scanning captures certain features of the patient or performs certain operations on the patient. The additional function is not merely a control function for controlling devices. The one other device may comprise an active device such as the curing light 402, the still or video camera 108, the microphone 406, the ultrasound device 416, the laser emitter device 420, or a high-energy light emitting device for detecting caries.

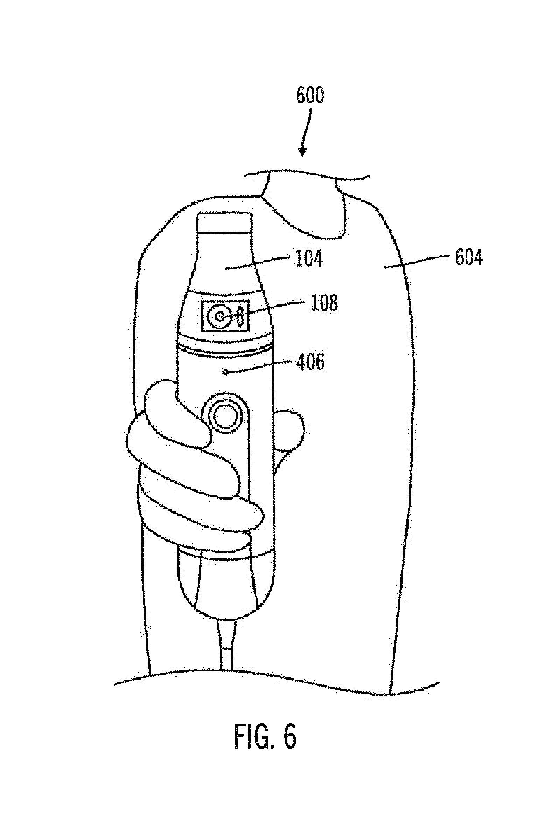

FIG. 6 illustrates a block diagram 600 that shows an operator 604 (e.g., a dental practitioner) holding the multifunction wand 104 via a handle located towards the proximal end of the multifunction wand 104, in accordance with certain embodiments. The camera 108 is shown facing away from the operator 604 to capture images of the face of patient.

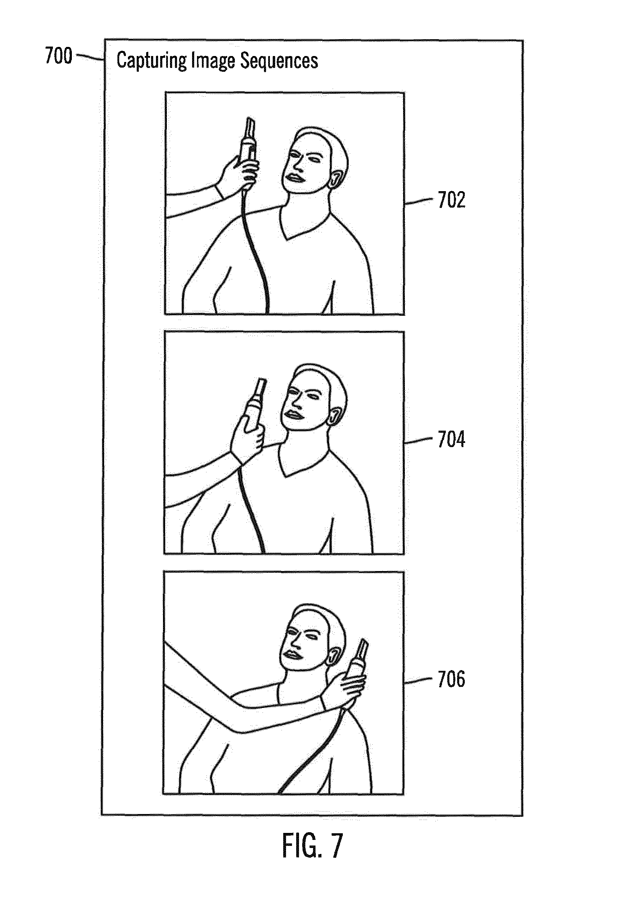

FIG. 7 illustrates a block diagram 700 in which an operator captures a sequence of images of a patient's face from various positions and orientations of the multifunction wand 104, in accordance with certain embodiments. In diagram 702 the operator captures a frontal view of the patient via the camera 108 and then captures another frontal view of the patient from another position as shown in diagram 704. Subsequently the operator may capture one or more lateral view of the patient via the camera 108 as shown via diagram 706.

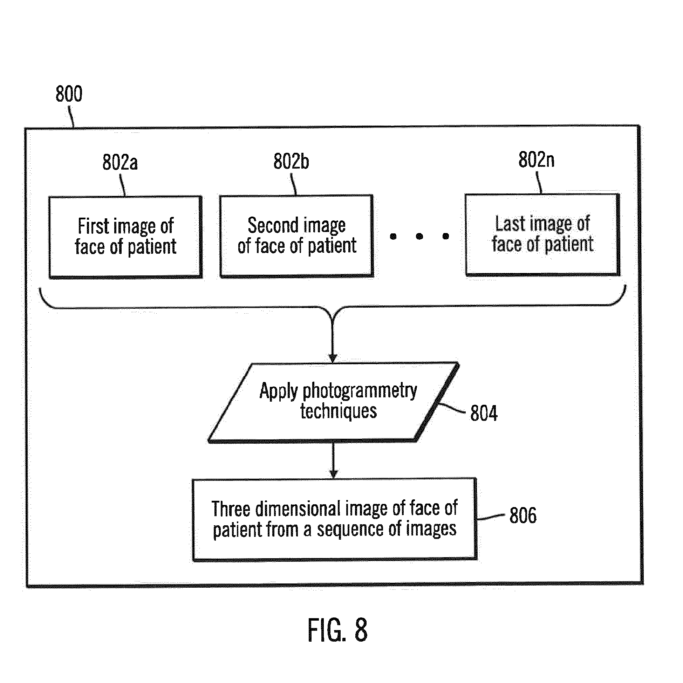

FIG. 8 illustrates a block diagram 800 that shows how three dimensional images of the face of a patient are generated via photogrammetry techniques, in accordance with certain embodiments. A sequence of images of a patient's face is captured via the camera 108 as shown in FIG. 7. The captured images are shown via reference numerals 802a, 802b,...802n. Photogrammetric techniques are applied (reference numeral 804) to generate 3D images 806 of the face of the patient.

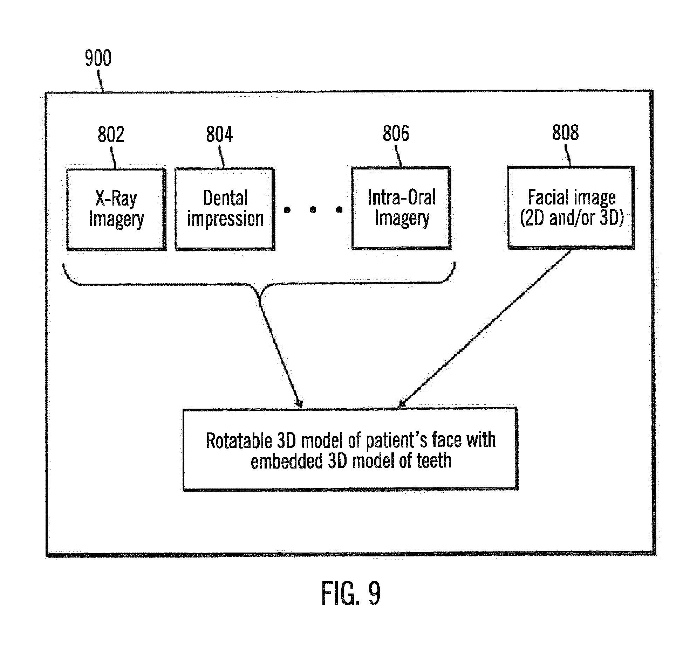

FIG. 9 illustrates a block diagram 900 that shows how a rotatable 3D model of a patient's face with embedded three-dimensional model of teeth is generated, in accordance with certain embodiments.

In certain embodiments, X-ray imagery 802 captured via X-ray machines, dental impressions 804, and intra-oral imagery 806 captured via the intra-oral camera 106 may be combined with the 2D and/or 3D facial images generated by using the camera 108 to generate rotatable 3D models of a patient's face with embedded 3D model of the patient's teeth and viewed on the display 112. Other images (e.g. ultrasound) may also be used to generate the rotatable 3D modes of the patient's face with embedded 3D model of the patient's teeth.

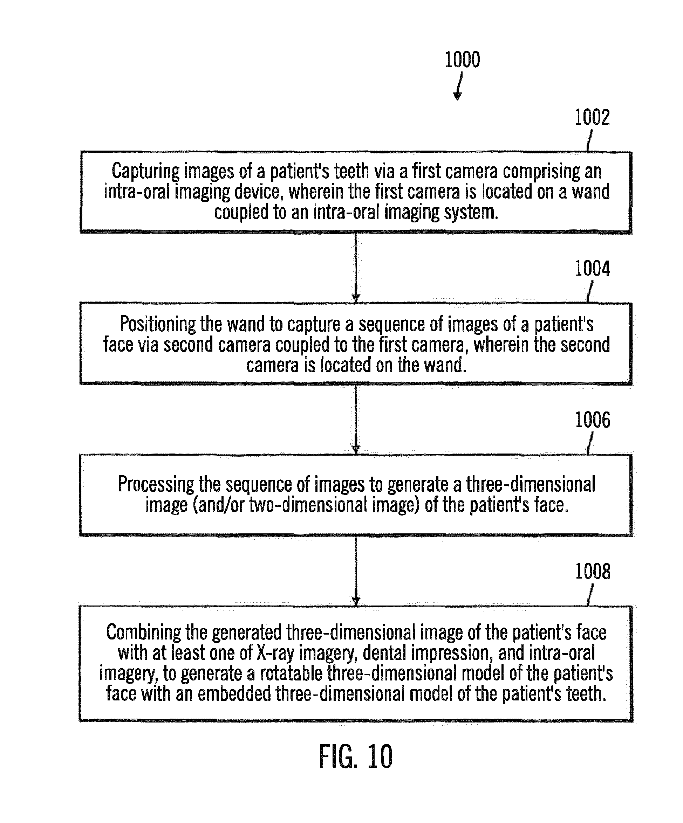

FIG. 10 illustrates exemplary operations performed in accordance with certain embodiments. The operations shown in FIG. 10 may be performed by the multifunction wand 104 of the intra-oral imaging system 102.

Control starts at block 1002, in which images of a patient's teeth are captured via a first camera 106 comprising an intra-oral imaging device, wherein the first camera 106 is located on a wand 104 coupled to an intra-oral imaging system 102.

Control proceeds to block 1004, in which the wand 104 is positioned to capture a sequence of images of a patient's face via second camera 108 (e.g., the still/video camera) coupled to the first camera, wherein the second camera 108 is located on the wand 104. The sequence of images are processed to generate (at block 1006) a three-dimensional image (and/or two-dimensional image) of the patient's face.

The generated three-dimensional image of the patient's face are combined with at least one of X-ray imagery, dental impression, and intra-oral imagery, to generate (at block 1008) a rotatable three-dimensional model of the patient's face with an embedded three-dimensional model of the patient's teeth.

Therefore FIGS. 1-10 illustrate certain embodiments in which a multifunction wand 104 is augmented with an intra-oral camera 106 for capturing intra-oral images of a patient's teeth, and a still or video camera for capturing a plurality images of a patient's face. The intra-oral images and the images of the patient's face are processed and combined to generate a rotatable three-dimensional model of the patient's face with an embedded three-dimensional model of the patient's teeth.

The operations described in FIGS. 1-10 may be implemented as a method, apparatus or computer program product using techniques to produce software, firmware, hardware, or any combination thereof. Additionally, certain embodiments may take the form of a computer program product embodied in one or more computer readable storage medium(s) having computer readable program code embodied therein.

A computer readable storage medium may include an electronic, magnetic, optical, electromagnetic, or semiconductor system, apparatus, or device, or any suitable combination of the foregoing. The computer readable storage medium may also comprise an electrical connection having one or more wires, a portable computer diskette or disk, a hard disk, a random access memory (RAM), a read-only memory (ROM), an erasable programmable read-only memory (EPROM or Flash memory), a portable compact disc read-only memory (CD-ROM), an optical storage device, a magnetic storage device, etc. A computer readable storage medium may be any tangible medium that can contain, or store a program for use by or in connection with an instruction execution system, apparatus, or device.

Computer program code for carrying out operations for aspects of the present invention may be written in any combination of one or more programming languages.

Aspects of the present invention are described below with reference to flowchart illustrations and/or block diagrams of methods, system and computer program products according to certain embodiments. At least certain operations that may have been illustrated in the figures show certain events occurring in a certain order. In alternative embodiments, certain operations may be performed in a different order, modified or removed. Additionally, operations may be added to the above described logic and still conform to the described embodiments. Further, operations described herein may occur sequentially or certain operations may be processed in parallel. Yet further, operations may be performed by a single processing unit or by distributed processing units. Computer program instructions can implement the blocks of the flowchart. These computer program instructions may be provided to a processor of a computer for execution.

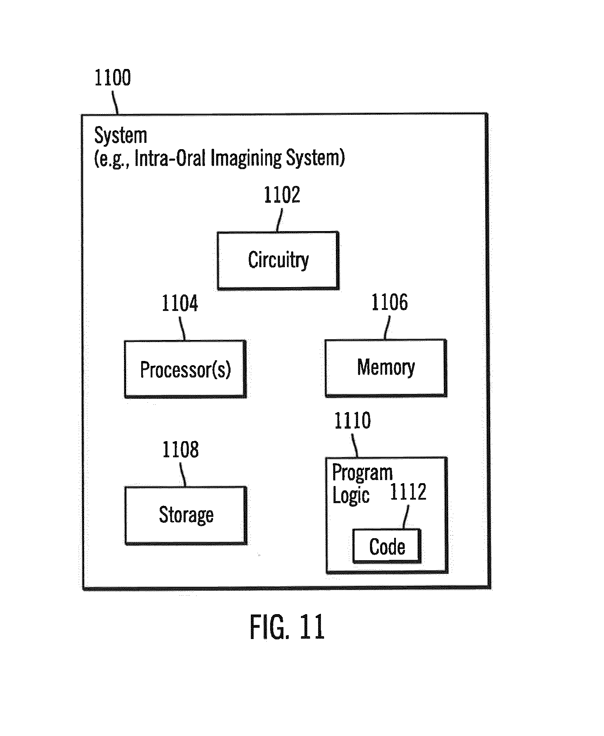

FIG. 11 illustrates a block diagram that shows certain elements that may be included in the intra-oral imaging system 102 or any of the computational devices 120, in accordance with certain embodiments. The system 1100 may comprise intra-oral imaging system 102 or the computational devices 120 and may include a circuitry 1102 that may in certain embodiments include at least a processor 1104, such as the processor 110. The system 1100 may also include a memory 1106 (e.g., a volatile memory device), and storage 1108. The storage 1108 may include a non-volatile memory device (e.g., EEPROM, ROM, PROM, RAM, DRAM, SRAM, flash, firmware, programmable logic, etc.), magnetic disk drive, optical disk drive, tape drive, etc. The storage 1108 may comprise an internal storage device, an attached storage device and/or a network accessible storage device. The system 1100 may include a program logic 1110 including code 1112 that may be loaded into the memory 1106 and executed by the processor 1104 or circuitry 1102. In certain embodiments, the program logic 1110 including code 1112 may be stored in the storage 1108. In certain other embodiments, the program logic 1110 may be implemented in the circuitry 1102. Therefore, while FIG. 11 shows the program logic 1110 separately from the other elements, the program logic 1110 may be implemented in the memory 1106 and/or the circuitry 1102.

The terms "an embodiment", "embodiment", "embodiments", "the embodiment", "the embodiments", "one or more embodiments", "some embodiments", and "one embodiment" mean "one or more (but not all) embodiments of the present invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

Devices that are in communication with each other need not be in continuous communication with each other, unless expressly specified otherwise. In addition, devices that are in communication with each other may communicate directly or indirectly through one or more intermediaries.

A description of an embodiment with several components in communication with each other does not imply that all such components are required. On the contrary a variety of optional components are described to illustrate the wide variety of possible embodiments.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features.

The foregoing description of various embodiments of the invention has been presented for the purposes of illustration and description. It is not intended to be exhaustive or to limit the invention to the precise form disclosed. Many modifications and variations are possible in light of the above teaching.

## Claims

1. A wand having a housing, wherein the wand is coupled to an intra-oral imaging system, the wand **characterized by** a first device coupled to the housing that is an intra-oral imaging device for capturing images of a patient's teeth, and a second device coupled to the housing to provide an additional function.

2. The wand of claim 1, wherein the second device is a camera for capturing images of the patient's face.

3. The wand of either claim 1 or claim 2, wherein the second device is a light emitting diode (LED) curing light for hardening sealants.

4. The wand of claim 3, wherein the LED curing light is positioned to emit light through a tip of the wand, wherein a curing light control is positioned on a handle of the wand, and wherein the curing light control controls operation of the LED curing light.

5. The wand of any preceding claim, wherein the second device is a microphone for capturing oral instructions of a patient or a dental practitioner.

6. The wand of any preceding claim, wherein the second device is an ultrasound device to capture ultrasound imagery of the patient's oral cavity.

7. The wand of any preceding claim, wherein the second device is a laser emitter device that generates photoacoustic waves greater than 1200 nanometers in wavelength to turbulently clean interiors of root and lateral canal systems or cause cell lysis and dissolution of inorganics in biotic systems.

8. The wand of any preceding claim, wherein the second device is a high-energy light emitting device for detecting caries.

9. The wand of claim 1, wherein the intra-oral imaging device is a first camera, and the second device is a second camera for capturing images of the patient's face, the wand further **characterized by** a tip, wherein the first camera for capturing the images of the patient's teeth is positioned to image through the tip, and a handle for holding the wand, wherein the second camera for capturing the images of the patient's face is positioned on the handle.

10. The wand of claim 9, the wand further comprising:
a camera control positioned on the handle, wherein the camera control controls operation of the second camera, and wherein the second camera is at least one of a still camera and a video camera.

11. The wand of claim 1, wherein the intra-oral imaging device is a first camera, and the second device is a second camera for capturing images of the patient's face, the wand further comprising:
a light emitting diode (LED) curing light for hardening sealants;
a microphone for capturing oral instructions of a patient or a dental practitioner;
an ultrasound device to capture ultrasound imagery of the patient's oral cavity;
a laser emitter device that generates photoacoustic waves greater than 1200 nanometers in wavelength to turbulently clean interiors of root and lateral canal systems or cause cell lysis and dissolution of inorganics in biotic systems; and
a high-energy light emitting device for detecting caries.

12. An intra-oral imaging system comprising a processor and the wand of any preceding claim, further **characterized in that** the wand is capable of transmitting data to the processor.

13. The intra-oral imaging system of claim 12, wherein the wand is positioned to capture a sequence of images of a patient's face, and wherein the sequence of images are processed by the processor to generate a two-dimensional image of the patient's face.

14. The intra-oral imaging system of claim 12, wherein the wand is positioned to capture a sequence of images of a patient's face, and wherein the sequence of images are processed by the processor to generate a three-dimensional image of the patient's face.

15. The intra-oral imaging system of claim 14, wherein the generated three-dimensional image of the patient's face are combined with at least one of X-ray imagery, dental impression, and intra-oral imagery, to generate a rotatable three-dimensional model of the patient's face with an embedded three-dimensional model of the patient's teeth.
